# EUROPÄISCHE PATENTSCHRIFT

(11) **EP 2 358 471 B1**
(45) Veröffentlichungstag und Bekanntmachung des Hinweises auf die Patenterteilung: **08.08.2012**
(21) Anmeldenummer: 09756670.7
(22) Anmeldetag: 06.11.2009
(51) Int. Cl.: B01J 31/24, B01J 31/40

(54) **VERFAHREN ZUR RÜCKGEWINNUNG VON RHODIUM AUS RHODIUMKOMPLEXVERBINDUNGEN ENTHALTENDEN WÄSSRIGEN LÖSUNGEN**
METHOD FOR RECLAIMING RHODIUM FROM AQUEOUS SOLUTIONS COMPRISING RHODIUM COMPLEX BONDS
PROCÉDÉ POUR LA RÉCUPÉRATION DU RHODIUM À PARTIR DE SOLUTIONS AQUEUSES CONTENANT DES COMPOSÉS COMPLEXES DU RHODIUM

(30) Priorität: 18.11.2008 DE 102008057857
(43) Veröffentlichungstag der Anmeldung: 24.08.2011
(73) Patentinhaber: OXEA GmbH, 46147 Oberhausen (DE)
(72) Erfinder: GREB, Wolfgang, 46535 Dinslaken (DE); KLEIN, Thomas, 46149 Oberhausen (DE); LUKAS, Rainer, 45133 Essen (DE); SCHMID, Klaus, 46539 Dinslaken (DE); ZGORZELSKI, Wolfgang, 46149 Oberhausen (DE); FISCHBACH, Andreas, 84419 Schwindegg (DE)
(86) Internationale Anmeldenummer: PCT/EP2009/007955
(87) Internationale Veröffentlichungsnummer: WO 2010/057582

(56) Entgegenhaltungen:
- EP-A- 0 255 673
- EP-A- 0 355 837
- EP-A- 0 367 957
- US-B1- 6 180 838

## Beschreibung

Die vorliegende Erfindung betrifft ein Verfahren zur Rückgewinnung von Rhodium aus Rhodiumkomplexverbindungen enthaltenden wässrigen Lösungen. Die Rhodiumkomplexverbindungen bilden zusammen mit überschüssig eingesetzten Komplexliganden unter anderem ein Katalysatorsystem, das - wie in der DE-PS 26 27 354 beschrieben - zur Hydroformylierung von Olefinen eingesetzt wird. Das Katalysatorsystem bildet sich unter Reaktionsbedingungen aus Rhodium und einem wasserlöslichen organischen Phosphin, das im Überschuss eingesetzt wird. Seine Wasserlöslichkeit beruht auf der Anwesenheit von Sulfonsäuregruppen, die in dem organischen Phosphin vorhanden sind. Der Phosphor-Ligand wird bevorzugt als Alkali-, Ammonium- oder Erdalkalisulfonat verwendet.

Bei längerem Gebrauch des Katalysatorsystems nimmt die Selektivität der Umsetzung ab. Diese Selektivitätsminderung ist zum einen auf das Einwirken von Katalysatorgiften, wie Eisencarbonyl, das sich durch Einwirken von Kohlenmonoxid auf die Wand des Reaktors bilden kann, und auf höhersiedende, sich aus den Aldehyden bildende Kondensationsprodukte und zum anderen auf die Verringerung des überschüssig eingesetzten sulfonierten Phosphins durch Oxidation zu Phosphinoxiden oder Abbau zu aromatischen Sulfonsäuren zurückzuführen. Daneben entstehen aus den Phosphinen durch die Reduktion von Sulfonsäuregruppen auch Phosphinsulfide.
Da weder Phosphinoxide noch Phosphinsulfide noch aromatische Sulfonsäuren im Hydroformylierungskatalysator erwünscht sind, ist es erforderlich, die verbrauchte Katalysatorlösung zu ersetzen. Aus Gründen der Wirtschaftlichkeit ist es notwendig, aus dieser Katalysatorlösung das Rhodium abzutrennen und zurückzugewinnen.

EP 0 255 673 A2 behandelt ein Verfahren zur Rückgewinnung von Rhodium aus Rhodiumkomplexverbindungen enthaltenden wässrigen Lösungen, die als Katalysatorphase bei der Hydroformylierung von Olefinen verwendet werden und die nach längerem Gebrauch keine zufriedenstellende Selektivität und Produktivität in der Hydroformylierungsreaktion mehr aufweisen. Diese wässrige Rhodiumkomplexverbindungen enthaltende Lösung wird zunächst mit dem Salz einer Carbonsäure im Überschuss, bezogen auf Rhodium, versetzt und anschließend mit einem Oxidationsmittel behandelt. Je g-Atom Rhodium werden der wässrigen Lösung 20 bis 500 und insbesondere 40 bis 300 mol Carbonsäuresalz zugesetzt. Im Verlauf der Oxidation bilden sich wasserunlösliche Rhodiumverbindungen, die sich als ölige Schicht von der wässrigen Phase abscheiden. Die abgeschiedenen Rhodiumverbindungen werden dann, gegebenenfalls nach Zugabe eines organischen, mit Wasser nicht mischbaren Lösungsmittels, durch einfache Phasentrennung entfernt.

Eine Weiterentwicklung des aus der EP 0 255 673 A2 bekannten Rhodiumrückgewinnungsverfahrens wird in der EP 0 367 957 A2 beschrieben. Nach dieser Arbeitsweise erfolgt der Oxidationsschritt mit Wasserstoffperoxid in Kombination mit Sauerstoff.

Nach den bekannten Verfahren liegt die Rhodiumrückgewinnungsrate bei 94 bis 98 % des ursprünglich vorhandenen Rhodiums. Das restliche Rhodium verbleibt in der wässrigen Lösung und der Stand der Technik erwähnt nur, dass diese Rhodiummenge gesondert zurückgewonnen werden kann, ohne näher auf die Rückgewinnungsmethode für diese Restmenge einzugehen. Die Rückgewinnung von Rhodium aus den Rhodium haltigen Stoffströmen, die bei der Hydroformylierungsreaktion oder Oxo-Synthese anfallen, mittels Ionenaustauscherharzen ist im Stand der Technik bekannt. Nach der in der WO 02/096555 A1 beschriebenen Ausführungsform wird die Hydroformylierungsreaktion in einer homogenen, organischen Flüssigkeit, die den gelösten Rhodium-Phosphin-Katalysator und überschüssiges Phosphin sowie Ausgangsolefin und gebildete Reaktionsprodukte enthält, durchgeführt. Der nach Aldehydabtrennung anfallende Rhodium haltige Stoffstrom wird aufkonzentriert, einer oxidativen Behandlung unterzogen und anschließend in einer weiteren Reaktionszone mit einem Gemisch aus Wasserstoff und Kohlenmonoxid in Gegenwart von Triphenylphosphin behandelt. Schließlich wird die so vorbehandelte Lösung auf ein saures Ionenaustauscherharz gegeben. Für die Bindung des Rhodiums auf dem Ionenaustauscherharz haben sich solche mit Sulfonsäuregruppen in der protonierten Form bewährt. Zur Rückgewinnung des Rhodiums wird das beladene Ionenaustauscherharz mit umzusetzendem Olefin, gelöst in einem geeigneten organischen Lösungsmittel, und einem Gemisch aus Kohlenmonoxid und Wasserstoff behandelt. Die Rhodium haltige, eluierte Flüssigkeit wird anschließend in den Hydroformylierungsreaktor zurückgeführt.

Ein weiteres Verfahren zur Rückgewinnung von Rhodium aus Produktströmen, die bei der Hydroformylierung von Olefinen in einer homogenen organischen Phase anfallen, ist aus WO 02/20451 A1 bekannt. Die Umsetzung erfolgt mit einem homogen gelösten, vorzugsweise nicht mit Liganden modifizierten Rhodiumkomplex als Katalysator. Rhodium wird aus den anfallenden Stoffströmen an einem basischen Ionenaustauscherharz gebunden, das Stickstoff haltige Reste als Ankergruppen enthält. Zur Rückgewinnung des Rhodiums wird das Ionenaustauscherharz verbrannt und Rhodium aus den Ascherückständen entfernt.

Auch nach der Lehre von DE 20 45 416 A1 erfolgt die Wiedergewinnung von Rhodium aus den organischen Produktströmen der Oxoreaktion mittels basischer Ionenaustauscherharze. Das in dem organischen Medium homogen gelöste, nicht mit Liganden modifizierte Rhodiumcarbonylat wird an einem basischen Ionenaustauscherharz gebunden. Zur Regenerierung wird das beladene Harz mit einem Gemisch aus niederen Alkanolen, Wasser und wasserlöslichen aliphatischen Aminen in Gegenwart von molekularem Sauerstoff behandelt. Das Rhodium haltige Eluat wird eingedampft und Rhodium zu einer für die Oxoreaktion geeigneten Form aufgearbeitet.

Für die Rückgewinnung von Rhodium mittels Ionenaustauscherharzen aus wässrigen Lösungen, die als Katalysatorphase bei der Hydroformylierung von Olefinen verwendet werden, gibt der Stand der Technik nur wenige Hinweise. Nach der Lehre der EP 0 355 837 A2 kann eine wässrige, Rhodium haltige Lösung mit einem basischen oder sauren Ionenaustauscherharz behandelt werden, um Rhodium zu binden. Das bekannte Verfahren erfordert jedoch zunächst, das Ionenaustauscherharz mit einer ionischen Organophosphorverbindung, beispielsweise mit Triphenylphosphinmonosulfonsäure, zu modifizieren. Durch Behandeln des beladenen Harzes mit einer Lösung, die eine Organophosphorverbindung, beispielsweise Triphenylphosphin, gelöst enthält, kann Rhodium von dem modifizierten Ionenaustauscherharz eluiert werden.

US 6,180,838 B1 betrifft die Herstellung von Alkandiolen durch Hydroformylierung eines Alkylenoxids in einem nicht mit Wasser mischbaren Lösungsmittel in Gegenwart nicht phosphinhaltiger Rhodiumkatalysatoren unter Bildung eines Hydroxyaldehyds. Das Reaktionsgemisch wird anschließend mit Wasser extrahiert, wobei der Hydroxyaldehyd in die wässrige Phase übergeht und der Rhodiumkomplex zum Teil in der organischen Phase verbleibt. Der wässrige Extrakt des Hydroformylierungsgemisches kann vor der Hydrierung zum Alkandiol mit einem Ionenaustauscher in Kontakt gebracht werden, um anwesendes Rhodium zu entfernen.

Aufgrund der hohen Kosten für Rhodium ist es wünschenswert, die restlichen Rhodiummengen aus den wässrigen Lösungen, die nach Abtrennung der wasserunlöslichen Rhodiumverbindungen nach den Oxidationsverfahren gemäß EP 0 255 673 A2 und EP 0 367 957 A2 anfallen, möglichst vollständig zurückzugewinnen. Die Rückgewinnung dieser Restmengen ist jedoch nicht unproblematisch, da die Rhodiumkonzentration in der angefallenen wässrigen Lösung nur noch sehr gering ist. Ebenfalls enthält die wässrige Lösung eine vergleichsweise hohe Salzkonzentration an sulfonierten Phosphinoxiden, die sich während des Oxidationsschritts durch den Zerfall des Rhodium-Phosphin-Komplexes und durch die Oxidation der im Überschuss vorhandenen sulfonierten Phosphine bilden. Zudem wird bei der oxidativen Behandlung ein wasserlösliches Salz einer Carbonsäure im Überschuss in Bezug auf Rhodium zugesetzt. Nach der Lehre von EP 0 255 673 A2 erschweren jedoch zu hohe Salzkonzentrationen die Rhodiumrückgewinnung.

Es besteht daher der Bedarf an einem Verfahren zur Rückgewinnung der Restmengen an Rhodium aus Rhodiumkomplexverbindungen enthaltenden wässrigen Lösungen, die als Katalysatorphase bei der Hydroformylierung von Olefinen verwendet werden und die anschließend einer oxidativen Behandlung unterzogen werden, auf eine einfache Weise.

Überraschenderweise wurde gefunden, dass aus der bei der oxidativen Rückgewinnung von Rhodium aus Rhodiumkomplexen anfallenden wässrigen Lösung, die als Katalysatorphase bei der Hydroformylierung von Olefinen verwendet wird und die wasserlösliche Rhodiumkomplexe enthält, Restmengen des Rhodiums zurückgewonnen werden können, wenn diese wässrige Lösung mit einem Ionenaustauscherharz behandelt wird.

Die Erfindung besteht daher in einem Verfahren zur Rückgewinnung von Rhodium aus Rhodiumkomplexverbindungen enthaltenden wässrigen Lösungen, die als Katalysatorphase bei der Hydroformylierung von Olefinen verwendet werden, bei dem man die wässrige Katalysatorlösung, die Phosphine der Formel 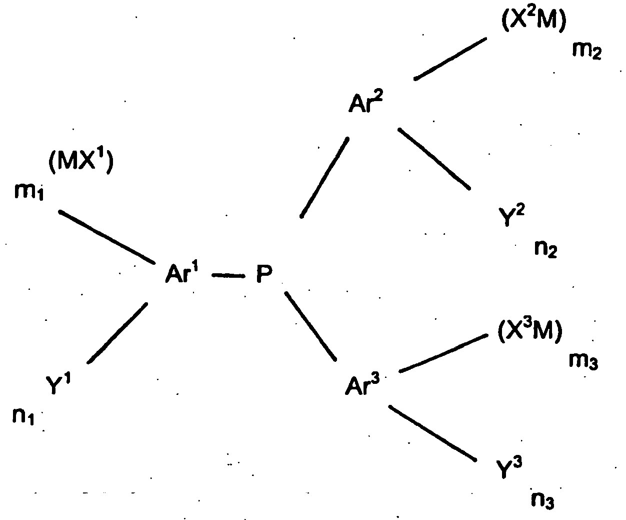 in der Ar¹, Ar² und Ar³ jeweils für eine Phenyl- oder Naphthylgruppe, Y¹, Y², Y³ jeweils für eine geradkettige oder verzweigte Alkylgruppe mit 1 bis 4 C-Atomen, eine Alkoxygruppe, ein Halogenatom, eine OH-, CN-, NO₂- oder - R¹R²N-Gruppe steht, in der R¹ und R² jeweils eine geradkettige oder verzweigte Alkylgruppe mit 1 bis 4 C-Atomen ist; X¹, X², X³ jeweils einen Carboxylat-(COO⁻) und/oder Sulfonat-(SO₃-)-Rest bedeutet, n₁, n₂, n₃ gleiche oder verschiedene ganze Zahlen von 0 bis 5 sind, M ein Alkalimetallion, das Äquivalent eines Erdalkalimetall- oder Zinkions oder ein Ammonium- oder quartäres Alkylammoniumion der allgemeinen Formel N(R³R⁴R⁵R⁶)⁺ darstellt, in der R³, R⁴, R⁵, R⁶ jeweils eine geradkettige oder verzweigte Alkylgruppe mit 1 bis 4 C-Atomen ist, und m₁, m₂, m₃ gleiche oder verschiedene ganze Zahlen von 0 bis 3 sind, wobei mindestens eine Zahl m₁, m₂ oder m₃ gleich oder größer 1 ist, als wasserlöslichen Komplexliganden enthält, mit einem Oxidationsmittel behandelt und das als in Wasser unlösliche Verbindung abgeschiedene Rhodium abtrennt. Es ist dadurch gekennzeichnet, dass man die nach Abtrennung der abgeschiedenen Rhodiumverbindung erhaltene wässrige Lösung mit einem funktionalisierten Ionenaustauscherharz behandelt, das Thioharnstoff-, Benzylamin-, Imidazol-, Thiol-, Mercaptophenylamino-, Aminoethylamino-, Aminoalkyl-, Mercaptoalkyl-, Alkylthioharnstoff oder Polyamin-Gruppen als Gruppen mit komplexbildendem Charakter enthält.

Überraschenderweise wurde gefunden, dass sich die in der wässrigen Lösung vorhandenen Restmengen an Rhodium durch Behandeln mit dem Ionenaustauscherharz wirksam auf dem Ionenaustauscherharz niederschlagen lassen. Die Behandlung erfolgt im Allgemeinen bei Normaldruck, obwohl die Anwendung höherer Drücke, beispielsweise bis 0,5 MPa auch möglich ist. Auch müssen keine besonderen Vorkehrungen, wie das Arbeiten unter Synthesegas getroffen oder ein zusätzliches, gezieltes Modifizieren der Ionenaustauscherharze mit Komplexbildnern, die das Rhodium binden, vorgenommen werden. Nach dem erfindungsgemäßen Verfahren wird die wässrige, Restmengen an Rhodium enthaltende Lösung unter Luft auf das kommerziell erhältliche Ionenaustauscherharz gegeben. Dabei kann das Ionenaustauscherharz mit der rhodiumhaltigen Lösung bei jeglicher Temperatur in Kontakt gebracht werden. Bei Temperaturen unterhalb von Raumtemperatur muss man jedoch mit verhältnismäßig langen Kontaktzeiten rechnen, um eine ausreichende Rhodiumabscheidung zu erzielen. Wählt man zu hohe Temperaturen, ist eine Schädigung des Ionenaustauscherharzes aufgrund seiner begrenzten Temperaturstabilität zu befürchten. Vorzugsweise arbeitet man bei Temperaturen von 60 bis 120°C, insbesondere von 60 bis 90°C, und bei Normaldruck. Dennoch ist das erfindungsgemäße Verfahren nicht auf das Einstellen eines bestimmten Temperaturbereichs beschränkt und die jeweilige Temperatur kann den individuellen Erfordernissen entsprechend eingestellt werden.

Als Ionenaustauscherharze werden funktionalisierte Ionenaustauscherharze verwendet, die als kommerzielle Produkte zur Verfügung stehen.

Solche funktionalisierte Ionenaustauscherharze basieren auf einem Polymergerüst, beispielsweise auf quervernetztem Polystyrol, an dem Gruppen gebunden sind, die keine ausgeprägten Säure/Base-Eigenschaften aufweisen sondern die komplexbildenden Charakter besitzen, ausgewählt aus Thioharnstoff-, Benzylamin-, Imidazol-, Thiol-, Mercaptophenylamino-, Aminoethylamino-, Aminoalkyl-, Mercaptoalkyl-, Alkylthioharnstoff- oder Polyamin-Gruppen. Als besonders gut geeignet haben sich Ionenaustauscherharze mit Thioharnstoff- und Alkylthioharnstoffgruppen mit C1 bis C5-Alkylresten erwiesen. Derartige Harze stehen beispielsweise unter der Bezeichnung Lewatit Monoplus kommerziell zur Verfügung.

Die dem Rhodiumrückgewinnungsverfahren zugeführten Lösungen stammen aus der oxidativen Aufarbeitung einer wässrigen Lösung, die als Katalysatorphase bei der Hydroformylierung von Olefinen verwendet wird. Das Arbeiten mit einer wässrigen Katalysatorlösung, die Rhodiumkomplexverbindungen und im Überschuss eingesetzte Komplexliganden gelöst enthält, ist beispielsweise aus DE 26 27 354 C1 bekannt und diese Prozessführung wird auch als zweiphasiges oder heterogenes Hydroformylierungsverfahren bezeichnet. Nach längerem Gebrauch zeigt die wässrige Katalysatorlösung keine zufriedenstellende Selektivität in der Hydroformylierungsreaktion mehr. Sie wird aus dem Prozess ausgeschleust und darin enthaltenes Rhodium wird zurückgewonnen, beispielsweise nach einem oxidativen Aufschluss, gemäß EP 0 255 673 A2 oder EP 0 367 957 A2. Nach Abtrennen der sich dabei abscheidenden wasserunlöslichen Rhodiumverbindungen wird eine wässrige Lösung erhalten, die noch gelöstes Rhodium in einer Restkonzentration üblicherweise von 2 bis 10 ppm enthält. Die Restkonzentration an gelöstem Rhodium kann dabei über einen breiten Bereich schwanken, weil der oxidative Aufschluss der gebrauchten wässrigen Katalysatorlösung nicht immer die optimalen Ergebnisse liefert und daher schwankende Restmengen an Rhodium in der wässrigen Lösung verbleiben.

Die nach dem oxidativen Aufschluss erhaltene wässrige Lösung besitzt eine vergleichsweise hohe Salzkonzentration, üblicherweise in einem Bereich von 5 bis 30 Gew.-%, berechnet als Trockensubstanz und bezogen auf die wässrige Lösung. Der Feststoff besteht im Wesentlichen aus wasserlöslichen Carbonsäuresalzen, die während der oxidativen Behandlung der gebrauchten, wässrigen Katalysatorlösung im Allgemeinen in einer Menge von 20 bis 500, insbesondere 40 bis 300 mol je g-Atom Rhodium zugesetzt werden, aus dem wasserlöslichen Komplexliganden sowie aus seinen wasserlöslichen, während der Hydroformylierungsreaktion gebildeten Abbau- und Umwandlungsprodukten, beispielsweise Phosphinsulfiden, und aufgrund der oxidativen Behandlung gebildeten Phosphinoxiden.

Bei den wasserlöslichen Komplexliganden handelt es sich um Phosphine der Formel

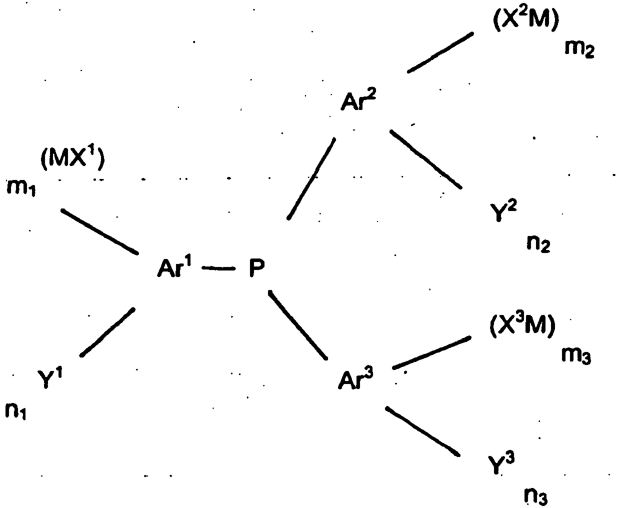

Hierin steht Ar¹, Ar² und Ar³ jeweils für eine Phenyl- oder Naphthylgruppe, Y¹, Y², Y³ jeweils für eine geradkettige oder verzweigte Alkylgruppe mit 1 bis 4 C-Atomen, eine Alkoxygruppe, ein Halogenatom, eine OH-, CN-, NO₂- oder R¹R²N-Gruppe, in der R¹ und R² jeweils eine geradkettige oder verzweigte Alkylgruppe mit 1 bis 4 C-Atomen ist; X¹, X², X³ bedeutet jeweils einen Carboxylat-(COO⁻) und/oder Sulfonat-(SO₃-)-Rest, n₁, n₂, n₃ sind gleiche oder verschiedene ganze Zahlen von 0 bis 5, M stellt ein Alkalimetallion, das Äquivalent eines Erdalkalimetall- oder Zinkions oder ein Ammonium- oder quartäres Alkylammoniumion der allgemeinen Formel N(R³R⁴R⁵R⁶)⁺ dar, in der R³, R⁴, R⁵, R⁶ jeweils eine geradkettige oder verzweigte Alkylgruppe mit 1 bis 4 C-Atomen ist, und m₁, m₂, m₃ sind gleiche oder verschiedene ganze Zahlen von 0 bis 3, wobei mindestens eine Zahl m₁, m₂ oder m₃ gleich oder größer 1 ist.

In der wässrigen Katalysatorlösung liegt neben dem am Rhodium komplexgebundenen Liganden auch noch überschüssiger Ligand vor. Durch den Oxidationsschritt wird nicht nur der im Überschuss vorhandene Komplexligand, sondern auch der Rhodiumkomplex selbst angegriffen, wobei auch der Ligand in die entsprechenden Phosphinoxide überführt wird und der ursprünglich vorhandene Rhodium-Komplex auseinanderfällt.

Neben den gelösten Salzen sind in der nach dem oxidativen Aufschluss erhaltenen wässrigen Lösung auch noch organische Bestandteile in geringen Mengen zugegen, beispielsweise höhersiedene Produkte wie Aldole, Kondensationspodukte und gegebenenfalls Lösungsvermittler, falls der wässrigen Katalysatorlösung zugesetzt, um in der Hydroformylierungsreaktion den Übergang der organischen Reaktanten in die Katalysatorlösung und den des wasserlöslichen Katalysatorsystems in die organische Phase zu begünstigen.

In einer besonderen Ausgestaltung des erfindungsgemäßen Verfahrens wird die bei dem oxidativen Aufschluss der gebrauchten wässrigen Katalysatorlösung sich abscheidende wasserunlösliche Rhodiumverbindung in einem organischen Lösungsmittel aufgenommen und wässrige und organische Phase voneinander getrennt. Geeignete organische Lösungsmittel sind Benzol, Toluol, Xylol, Cyclohexan, aliphatische Carbonsäuren und Carbonsäureester, aliphatische und cycloaliphatische Ketone mit 5 bis 10 Kohlenstoffatomen. Besonders geeignet sind Toluol und Xylol. Anschließend wird die organische Phase mit einer wässrigen, verdünnten Säure gewaschen, wobei das Volumenverhältnis zwischen organischer Phase und wässriger, verdünnter Säure zweckmäßigerweise so gewählt wird, dass die Phasenseparation unproblematisch erfolgt. Nach der Wäsche mit der wässrigen, verdünnten Säure wird die abgetrennte organische Phase nochmals mit Wasser, üblicherweise mit der gleichen Menge, gewaschen.

Für die Säurewäsche der organischen Phase eignen sich anorganische Säuren wie Salzsäure, Salpetersäure, Schwefelsäure oder Phosphorsäure sowie wasserlösliche organische Säuren mit 1 bis 5 Kohlenstoffatomen, wie Ameisensäure, Essigsäure, Propionsäure, die verschiedenen Buttersäuren und Pentansäuren. Üblicherweise besitzt die eingesetzte verdünnte Waschsäure einen Säuregehalt von 5 bis 25 Gew.-%. Vorzugsweise wird verdünnte Schwefelsäure mit einer Konzentration von 5 bis 25 Gew.-% für die Wäsche der organischen Phase verwendet.

Die angefallenen wässrigen Lösungen, bei denen es sich um die nach dem oxidativen Aufschluss der gebrauchten wässrigen Katalysatorlösung erhaltene wässrige Lösung sowie gegebenenfalls um die saure, wässrige Waschlösung aus der Säurewäsche der sich bei der oxidativen Behandlung abscheidenden wasserunlöslichen und in einem organischen Lösungsmittel aufgenommenen Rhodiumverbindungen sowie um das Waschwasser aus der anschließenden Wasserwäsche der organischen Phase handelt, werden getrennt oder in jeglicher Kombination vereint mit dem Ionenaustauscherharz behandelt. Vorzugsweise werden sämtliche wässrige Phasen vereinigt und anschließend mit dem Ionenaustauscherharz in Kontakt gebracht. Besonders geeignete Ionenaustauscherharze sind mit Thioharnstoff- oder Alkylthioharnstoffgruppen mit C1-C5-Alkylresten funktionalisierte Harze.

Zur praktischen Durchführung der Abtrennung der Restmengen an Rhodium, die in der wässrigen Lösung noch enthalten sind, bringt man die wässrige Lösung in einem geeigneten Reaktor mit dem Ionenaustauscherharz in Kontakt. Das Ionenaustauscherharz kann zum Beispiel in einem Rohrreaktor als Festbett angeordnet werden, durch das die rhodiumhaltige Flüssigkeit strömt. Das Festbettvolumen und die Größe der lonenaustauscherharzpartikel können in weiten Bereichen variiert und so den gewählten Reaktionsbedingungen und den Verfahrensgegebenheiten, wie der gewünschten Strömungsgeschwindigkeit, angepasst werden. Es hat sich bewährt, Raumgeschwindigkeiten im Bereich von 0,1 bis 2,5, insbesondere von 0,5 bis 1,5 (V wässr. Lösung /[V_{Ionenaustauscherharz}. h]) einzuhalten. Es handelt sich hierbei um Richtgrößen, die zweckmäßig einzuhalten sind. Bei geringeren Raumgeschwindigkeiten verbessert sich zwar die Rhodiumabscheidung aber der Abscheidungsprozess selbst dauert unverhältnismäßig lange. Bei höheren Raumgeschwindigkeiten wird der Abscheidungsprozess zwar in einem kürzeren Zeitraum bewältigt, die Abscheidung von Rhodium ist dann jedoch nicht vollständig.

Nach einer anderen Ausführungsform der erfindungsgemäßen Arbeitsweise suspendiert man das Ionenaustauscherharz, das in diesem Fall sehr feinteilig sein kann, in der rhodiumhaltigen wässrigen Lösung. Es ist zweckmäßig, die Suspension ständig zu bewegen, zum Beispiel durch Rühren oder Einleiten eines Gases, beispielsweise Luft oder Stickstoff, um den innigen Kontakt zwischen der flüssigen Phase und dem Ionenaustauscherharz zu erzielen. Das Massenverhältnis von flüssiger Phase zu Ionenaustauscherharz kann weitgehend frei und somit den individuellen Erfordernissen entsprechend eingestellt werden. Es hat sich bewährt, je 100 Gew.-teile rhodiumhaltiger wässriger Lösung 1 bis 10, vorzugsweise 3 bis 8 Gew.-teile Ionenaustauscherharz einzusetzen. Für die Durchführung dieser Verfahrensvariante eignen sich zum Beispiel Rührkessel oder Autoklaven.

Bei dieser Arbeitsweise unterliegt das Ionenaustauscherharz jedoch einer mechanischen Belastung und für die Vermischung von wässriger Phase mit dem Ionenaustauscherharz sind die Bedingungen so einzustellen, dass ein Abrieb an der Oberfläche der Harzteilchen oder gar eine mechanische Schädigung der Harzteilchen vermieden wird.

Die wässrige Lösung kann rezirkuliert werden, um durch mehrfache Behandlung der wässrigen Phase die Rhodiumabtrennung zu vervollständigen. Ebenso ist es möglich, die Adsorption in mehreren Stufen durchzuführen, sowohl absatzweise als auch kontinuierliche Reaktionsführung ist möglich.

Das neue Verfahren hat sich ausgezeichnet zur Rückgewinnung der Restmengen von Rhodium, die nach dem oxidativen Aufschluss der gebrauchten wässrigen Katalysatorlösung und nach Abtrennen unlöslicher Rhodiumverbindungen in der wässrigen Lösung verbleiben, bewährt. Falls die wasserunlöslichen Rhodiumverbindungen in einem organischen Lösungsmittel aufgenommen werden und die erhaltene organische Phase noch zusätzlich einer Wäsche mit einer wässrigen, verdünnten Säure und einer anschließenden Wasserwäsche unterzogen wird, können die Rhodiummengen, die hierbei in die wässrige Phase ausgetragen werden, ebenfalls durch das neue Verfahren zurückgewonnen werden. Es ist einfach durchzuführen und besonders bemerkenswert ist, dass es den Einsatz einer bereits aufgearbeiteten, wässrigen, gebrauchten Katalysatorlösung erlaubt, die auf der einen Seite nur noch geringe Mengen an Rhodium enthält aber auf der anderen Seite mit einer hohen Salzfracht beladen ist. Bis zu etwa 98% des Rhodiums im Einsatzprodukt können auf dem Ionenaustauscherharz abgeschieden werden und die Rhodiumkonzentration im behandelten Substrat kann bis unter 0,1 ppm gesenkt werden.

Weiterhin können in der gebrauchten, wässrigen Katalysatorlösung gelöste Schwermetallverbindungen, wie Verbindungen des Eisens, Kobalts, Nickels oder Chroms auf dem Ionenaustauscherharz niedergeschlagen werden. Die nach der erfindungsgemäßen Behandlung angefallenen wässrigen Lösungen weisen daher nur noch einen sehr geringen Schwermetallgehalt auf und können auf einfache Weise entsorgt werden.

Besonders geeignet ist das neue Verfahren für die Aufarbeitung von wässrigen Lösungen, die aus der Hydroformylierungsreaktion von Propylen, Buten-1 oder Gemischen enthaltend Buten-1 und Buten-2 unter Verwendung einer wässrigen Katalysatorlösung und nachfolgendem oxidativen Aufschluss der gebrauchten wässrigen Katalysatorlösung anfallen.

Besonders einfach gestaltet sich die Rückgewinnung des Rhodiums in metallischer oder oxidischer Form, bei der das beladene Ionenaustauscherharz verbrannt wird und das Edelmetall in elementarer Form oder als Oxid zurückbleibt.

Ebenfalls kann das mit Rhodium beladene Ionenaustauscherharz mit einer wässrigen Lösung enthaltend wasserlösliche Phosphine der Formel, 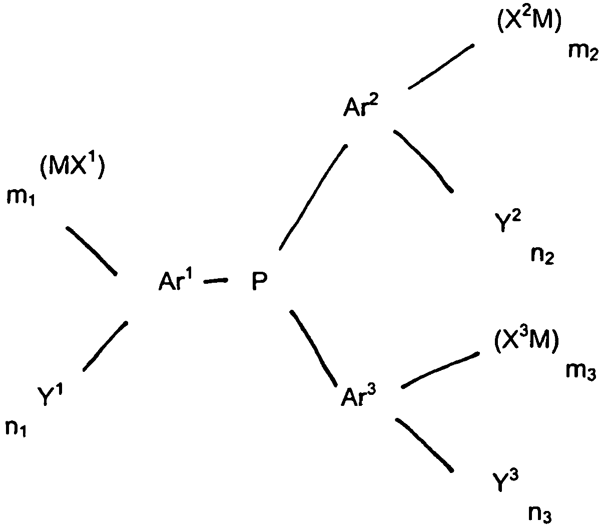 in der die Substituenten die zuvor genannte Bedeutung besitzen, in Gegenwart von Synthesegas, also einem Gemisch aus Kohlenmonoxid und Wasserstoff, bei erhöhter Temperatur und erhöhtem Druck in Kontakt gebracht werden. Dabei wird eine wässrige, Rhodium haltige Lösung zurückgewonnen, die beispielsweise in dem nach DE 26 27 354 C1 bekannten heterogenen Hydroformylierungsverfahren als Katalysatorlösung wiedereingesetzt werden kann.

In den folgenden Beispielen wird das erfindungsgemäße Verfahren näher beschrieben. Es ist selbstverständlich nicht auf die wiedergegebenen Ausführungsformen beschränkt.

### Allgemeine Versuchsdurchführung:

In einem 250 ml Dreihalskolben mit Rückflusskühler und Innenthermometer wurden 250 mg wasserfeuchtes Harz der kommerziell erhältlichen Type Lewatit^{®} Monoplus TP214 der Firma Lanxess eingewogen und 50 g der Rhodium haltigen, wässrigen Lösung zugegeben. Die Suspension wurde unter vorsichtigem Rühren auf 90°C erhitzt und bei dieser Temperatur 8 Stunden gerührt. Anschließend wurde der Restgehalt an Rhodium in der wässrigen Lösung mit der GAAS-Methode (Graphitrohratomabsorption) bestimmt.

In der nachfolgenden Tabelle 1 sind die Einsatzprodukte, ihre Rhodiumgehalte sowie die beobachteten Rhodiumrückgewinnungsquoten wiedergegeben. Das Abwasser (A) fällt nach dem oxidativen Aufschluss der gebrauchten wässrigen Katalysatorlösung an, beispielsweise durchgeführt nach den aus EP 0 255 673 A2 oder EP 0 367 957 A2 bekannten Verfahren. Sich abscheidende wasserunlösliche Rhodiumverbindungen werden mit Toluol in einer Menge, die ein Fünftel der wässrigen Phase entspricht, aufgenommen. Die schwefelsaure Waschlösung (B) wird durch Wäsche der toluolischen Phase mit der gleichen Mengen einer 10 gew.-%igen, wässrigen Schwefelsäure erhalten und das Waschwasser (C) fällt bei der anschließenden Wäsche der mit Schwefelsäure gewaschenen toluolischen Phase mit der gleichen Wassermenge an.

Bei dem Wiedereinsatz der vereinigten wässrigen Lösungen (A), (B) und (C) wird nahezu die gesamte, im Einsatz vorhandene Rhodiummenge zurückgewonnen.

**Tabelle 1: Abtrennung von Rhodium aus wässrigen Lösungen mittels Ionenaustauscherharz**

| **Einsatz** | **Rh Einsatz** | **Rh Einsatz** | **Rest-Rh (Verlust)** | **Rh ads. (berechnet)** | **Rh-Quote** |
|---|---|---|---|---|---|
| | **[ppm]** | **[mg]** | **[ppm]** | **[mg]** | **[%]** |
| Abwasser nach oxidativem Aufschluss (A) | 3,6 | 0,18 | 0,5 | 0,16 | 86% |
| Schwefelsaure Wäsche aus der toluolischen Phase (B) | 29 | 1,45 | 0,6 | 1,42 | 98% |
| Waschwasser der toluolischen Phase nach Schwefelsäurewäsche (C) | 3,2 | 0,16 | <0,1 | 0,16 | >99% |
| Vereinigte wässrige Lösungen (A)+(B)+(C) | 3,8 | 0,20 | 0,4 | 0,17 | 89% |
| Wiedereinsatz der vereinigten wässrigen Lösungen (A)+(B)+(C) mit frischem Ionenaustauscherharz | 0,4 | 0,02 | <0,1 | 0,02 | >99% |

## Patentansprüche

1. Verfahren zur Rückgewinnung von Rhodium aus Rhodiumkomplexverbindungen enthaltenden wässrigen Lösungen, die als Katalysatorphase bei der Hydroformylierung von Olefinen verwendet werden, bei dem man die wässrige Katalysatorlösung, die Phosphine der Formel 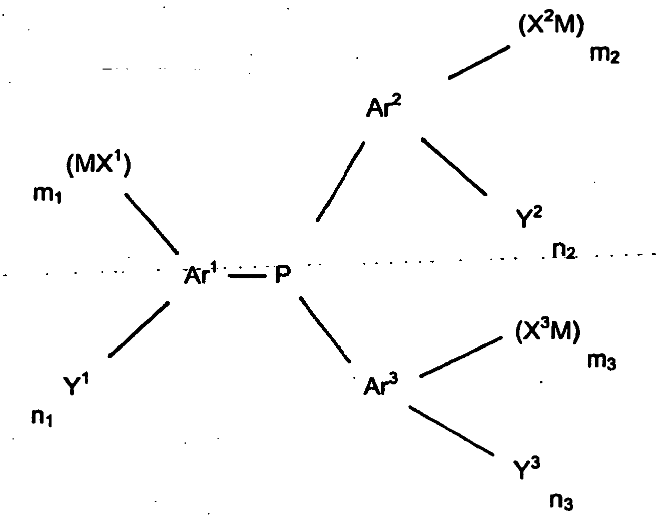 in der Ar¹, Ar² und Ar³ jeweils für eine Phenyl- oder Naphthylgruppe, Y¹, Y², Y³ jeweils für eine geradkettige oder verzweigte Alkylgruppe mit 1 bis 4 C-Atomen, eine Alkoxygruppe, ein Halogenatom, eine OH-, CN-, NO₂- oder R¹R²N-Gruppe steht, in der R¹ und R² jeweils eine geradkettige oder verzweigte Alkylgruppe mit 1 bis 4 C-Atomen ist; X¹, X², X³ jeweils einen Carboxylat-(COO⁻) und/oder Sulfonat-(SO₃-)-Rest bedeutet, n₁, n₂, n₃ gleiche oder verschiedene ganze Zahlen von 0 bis 5 sind, M ein Alkalimetallion, das Äquivalent eines Erdalkalimetall- oder Zinkions oder ein Ammonium- oder quartäres Alkylammoniumion der allgemeinen Formel N(R³R⁴R⁵R⁶)⁺ darstellt, in der R³, R⁴, R⁵, R⁶ jeweils eine geradkettige oder verzweigte Alkylgruppe mit 1 bis 4 C-Atomen ist, und m₁, m₂, m₃ gleiche oder verschiedene ganze Zahlen von 0 bis 3 sind, wobei mindestens eine Zahl m₁, m₂ oder m₃ gleich oder größer 1 ist, als wasserlöslichen Komplexliganden enthält, mit einem Oxidationsmittel behandelt und das als in Wasser unlösliche Verbindung abgeschiedene Rhodium abtrennt, **dadurch gekennzeichnet, dass** man die nach Abtrennung der abgeschiedenen Rhodiumverbindung erhaltene wässrige Lösung mit einem funktionalisierten Ionenaustauscherharz behandelt, das Thioharnstoff-, Benzylamin-, Imidazol-, Thiol-, Mercaptophenylamino-, Aminoethylamino-, Aminoalkyl-, Mercaptoalkyl-, Alkylthioharnstoff- oder Polyamin-Gruppen als Gruppen mit komplexbildendem Charakter enthält.

2. Verfahren gemäß Anspruch 1, **dadurch gekennzeichnet, dass** man der wässrigen Katalysatorlösung bei der Behandlung mit einem Oxidationsmittel ein wasserlösliches Carbonsäuresalz in einer Menge von 20 bis 500 mol je g-Atom Rhodium zusetzt.

3. Verfahren gemäß Anspruch 1 oder 2, **dadurch gekennzeichnet, dass** man das als in Wasser unlösliche Verbindung abgeschiedene Rhodium in einem organischen Lösungsmittel aufnimmt und die wässrige und organische Phase voneinander trennt, die organische Phase mit einer wässrigen, verdünnten Säure wäscht, und die abgetrennte organische Phase mit Wasser wäscht und dass man die angefallenen wässrigen Lösungen getrennt oder in jeglicher Kombination vereint mit dem Ionenaustauscherharz behandelt.

4. Verfahren gemäß einem oder mehreren der Ansprüche 1 bis 3, **dadurch gekennzeichnet, dass** man die Behandlung mit dem Ionenaustauscherharz bei Temperaturen von 60 bis 120°C, vorzugsweise 60 bis 90°C vornimmt.

5. Verfahren gemäß einem oder mehreren der Ansprüche 1 bis 4, **dadurch gekennzeichnet, dass** man die Behandlung mit dem Ionenaustauscherharz von Normaldruck bis 0,5 MPa, vorzugsweise bei Normaldruck vornimmt.

6. Verfahren gemäß Anspruch 1, **dadurch gekennzeichnet, dass** die funktionalisierten Ionenaustauscherharze Thioharnstoff- oder Alkylthioharnstoffgruppen mit C1-C5-Alkylresten enthalten.

7. Verfahren gemäß einem oder mehreren der Ansprüche 1 bis 6, **dadurch gekennzeichnet, dass** das Ionenaustauscherharz nach Behandlung verbrannt wird und Rhodium in metallischer oder oxidischer Form zurückgewonnen wird.

8. Verfahren gemäß einem oder mehreren der Ansprüche 1 bis 6, **dadurch gekennzeichnet, dass** das Ionenaustauscherharz nach Behandlung mit einer wässrigen Lösung enthaltend wasserlösliche Phosphine der Formel 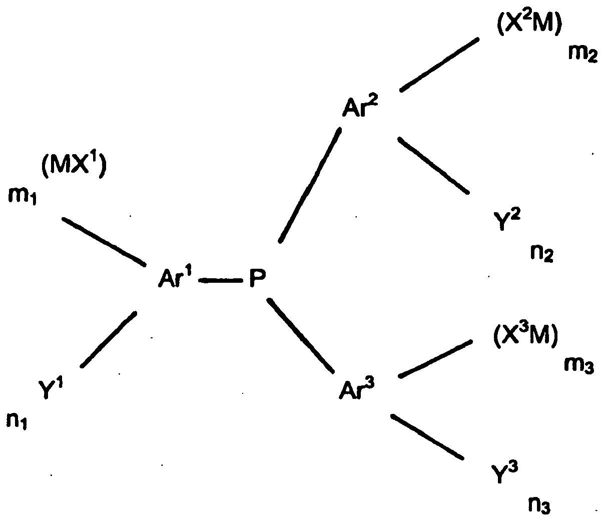 in der Ar¹, Ar² und Ar³ jeweils für eine Phenyl- oder Naphthylgruppe, Y¹, Y², Y³ jeweils für eine geradkettige oder verzweigte Alkylgruppe mit 1 bis 4 C-Atomen, eine Alkoxygruppe, ein Halogenatom, eine OH-, CN-, NO₂- oder R¹R²N-Gruppe steht, in der R¹ und R² jeweils eine geradkettige oder verzweigte Alkylgruppe mit 1 bis 4 C-Atomen ist; X¹, X², X³ jeweils einen Carboxylat-(COO⁻) und/oder Sulfonat-(SO₃-)-Rest bedeutet, n₁, n₂, n₃ gleiche oder verschiedene ganze Zahlen von 0 bis 5 sind, M ein Alkalimetallion, das Äquivalent eines Erdalkalimetall- oder Zinkions oder ein Ammonium- oder quartäres Alkylammoniumion der allgemeinen Formel N(R³R⁴R⁵R⁶)⁺ darstellt, in der R³, R⁴, R⁵, R⁶ jeweils eine geradkettige oder verzweigte Alkylgruppe mit 1 bis 4 C-Atomen ist, und m₁, m₂, m₃ gleiche oder verschiedene ganze Zahlen von 0 bis 3 sind, wobei mindestens eine Zahl m₁, m₂ oder m₃ gleich oder größer 1 ist, in Gegenwart von Synthesegas bei erhöhter Temperatur und erhöhtem Druck in Kontakt gebracht wird und eine wässrige, Rhodium haltige Lösung zurückgewonnen wird.

## Claims

1. A process for recovering rhodium from aqueous solutions containing rhodium complexes which are used as catalyst phase in the hydroformylation of olefins, in which the aqueous catalyst solution, which contains phosphines of the formula 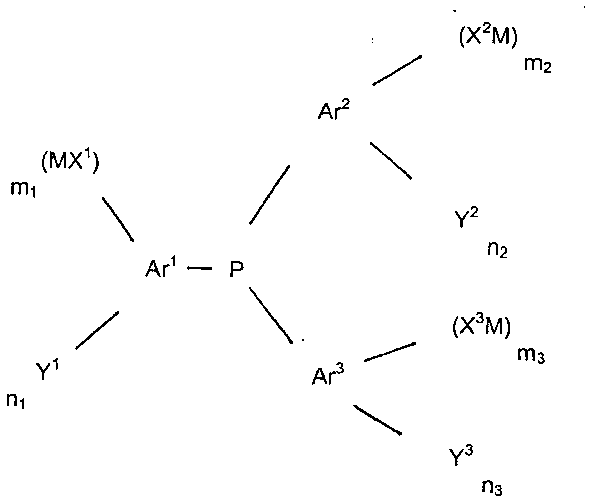 where Ar¹, Ar² and Ar³ are each a phenyl or naphthyl group, Y¹, Y², Y³ are each a straight-chain or branched alkyl group having from 1 to 4 carbon atoms, an alkoxy group, a halogen atom, a OH, CN, NO₂ or R¹R²N group, where R¹ and R² are each a straight-chain or branched alkyl group having from 1 to 4 carbon atoms; X¹, X², X³ are each a carboxylate (COO⁻) and/or sulfonate (SO₃⁻) radical, n₁, n₂, n₃ are identical or different integers from 0 to 5, M is an alkali metal ion, the equivalent of an alkaline earth metal ion or zinc ion or an ammonium or quaternary alkylammonium ion of the formula N(R³R⁴R⁵R⁶)⁺, where R³, R⁴, R⁵, R⁶ are each a straight-chain or branched alkyl group having from 1 to 4 carbon atoms, and m₁, m₂, m₃ are identical or different integers from 0 to 3, with at least one of the indices m₁, m₂ and m₃ being equal to or greater than 1, as water-soluble complex ligands, is treated with an oxidant and the rhodium which has separated out as a water-insoluble compound is separated off, wherein the aqueous solution obtained after the rhodium compound which has separated out has been separated off is treated with a functionalized ion exchange resin which contains thiourea, benzylamine, imidazole, thiol, mercaptophenylamino, aminoethylamino, aminoalkyl, mercaptoalkyl, alkylthiourea or polyamine groups as groups having complexing character.

2. The process as claimed in claim 1, wherein a water-soluble carboxylic acid salt is added in an amount of from 20 to 500 mol per gram atom of rhodium to the aqueous catalyst solution in the treatment with an oxidant.

3. The process as claimed in claim 1 or 2, wherein the rhodium which has separated out as a water-insoluble compound is taken up in an organic solvent and the aqueous and organic phases are separated from one another, the organic phase is washed with an aqueous, dilute acid and the organic phase which has been separated off is washed with water and the aqueous solutions obtained are treated, either separately or combined in any combination, with the ion exchange resin.

4. The process as claimed in one or more of claims 1 to 3, wherein the treatment with the ion exchange resin is carried out at temperatures of from 60 to 120°C, preferably from 60 to 90°C.

5. The process as claimed in one or more of claims 1 to 4, wherein the treatment with the ion exchange resin is carried out at from atmospheric pressure to 0.5 MPa, preferably at atmospheric pressure.

6. The process as claimed in claim 1, wherein the functionalized ion exchange resins contain thiourea groups or alkylthiourea groups having C1-C5-alkyl radicals.

7. The process as claimed in one or more of claims 1 to 6, wherein the ion exchange resin is burnt after treatment and rhodium is recovered in metallic or oxidic form.

8. The process as claimed in one or more of claims 1 to 6, wherein the ion exchange resin after treatment is brought into contact with an aqueous solution containing water-soluble phosphines of the formula 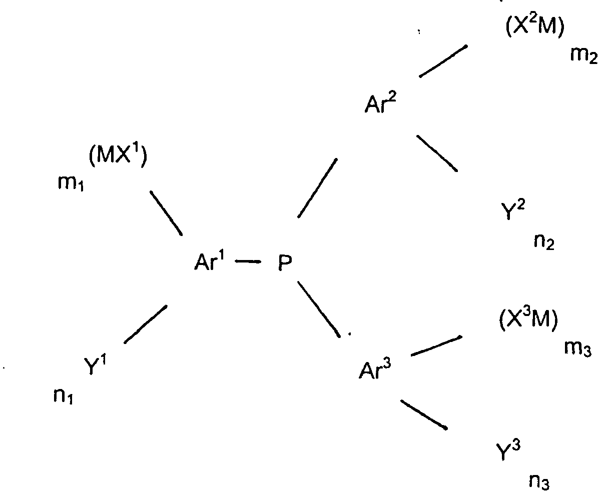 where Ar¹, Ar² and Ar³ are each a phenyl or naphthyl group, Y¹, Y², Y³ are each a straight-chain or branched alkyl group having from 1 to 4 carbon atoms, an alkoxy group, a halogen atom, a OH, CN, NO₂ or R¹R²N group, where R¹ and R² are each a straight-chain or branched alkyl group having from 1 to 4 carbon atoms; X¹, X², X³ are each a carboxylate (COO⁻) and/or sulfonate (SO₃⁻) radical, n₁, n₂, n₃ are identical or different integers from 0 to 5, M is an alkali metal ion, the equivalent of an alkaline earth metal ion or zinc ion or an ammonium or quaternary alkylammonium ion of the formula N(R³R⁴R⁵R⁶)⁺, where R³, R⁴, R⁵, R⁶ are each a straight-chain or branched alkyl group having from 1 to 4 carbon atoms, and m₁, m₂, m₃ are identical or different integers from 0 to 3, with at least one of the indices m₁, m₂ and m₃ being equal to or greater than 1, in the presence of synthesis gas at elevated temperature and elevated pressure and an aqueous, rhodium-containing solution is recovered.

## Revendications

1. Procédé de récupération de rhodium à partir de solutions aqueuses contenant des composés complexes de rhodium, qui sont utilisées en tant que phase catalytique lors de l'hydroformylation d'oléfines, selon lequel la solution catalytique aqueuse, qui contient des phosphines de formule 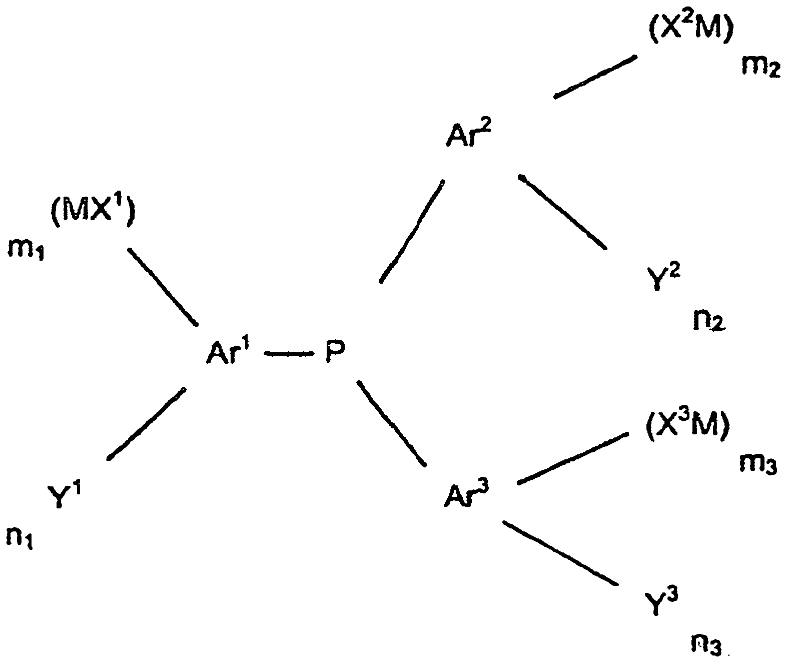 dans laquelle Ar¹, Ar² et Ar³ représentent chacun un groupe phényle ou naphtyle, Y¹, Y², Y³ représentent chacun un groupe alkyle linéaire ou ramifié de 1 à 4 atomes C, un groupe alcoxy, un atome d'halogène, un groupe OH, CN, NO₂ ou R¹R²N, dans lequel R¹ et R² sont chacun un groupe alkyle linéaire ou ramifié de 1 à 4 atomes C ; X¹, X², X³ signifient chacun un radical carboxylate (COO⁻) et/ou sulfonate (SO₃⁻), n₁, n₂, n₃ sont des nombres entiers identiques ou différents de 0 à 5, M représente un ion de métal alcalin, l'équivalent d'un ion de métal alcalino-terreux ou de zinc ou un ion d'ammonium ou d'alkylammonium quaternaire de formule générale N(R³R⁴R⁵R⁶)⁺, dans laquelle R³, R⁴, R⁵, R⁶ sont chacun un groupe alkyle linéaire ou ramifié de 1 à 4 atomes C, et m₁, m₂, m₃ sont des nombres entiers identiques ou différents de 0 à 3, au moins un nombre m₁, m₂ ou m₃ étant supérieur ou égal à 1, en tant que ligands complexes solubles dans l'eau, est traitée avec un oxydant et le rhodium précipité sous la forme d'un composé insoluble dans l'eau est séparé, **caractérisé en ce que** la solution aqueuse obtenue après la séparation du composé de rhodium précipité est traitée avec une résine échangeuse d'ions fonctionnalisée qui contient des groupes thiourée, benzylamine, imidazole, thiol, mercaptophénylamino, aminoéthylamino, aminoalkyle, mercaptoalkyle, alkylthiourée ou polyamine en tant que groupes à caractère complexant.

2. Procédé selon la revendication 1, **caractérisé en ce qu'**un sel d'acide carboxylique soluble dans l'eau est ajouté à la solution catalytique aqueuse lors du traitement avec un oxydant en une quantité de 20 à 500 moles par atome-gramme de rhodium.

3. Procédé selon la revendication 1 ou 2, **caractérisé en ce que** le rhodium précipité sous la forme d'un composé insoluble dans l'eau est absorbé dans un solvant organique, et la phase aqueuse et la phase organique sont séparées l'une de l'autre, la phase organique est lavée avec un acide aqueux dilué, et la phase organique séparée est lavée avec de l'eau, et **en ce que** les solutions aqueuses formées sont traitées avec la résine échange d'ions séparément ou réunies selon une combinaison quelconque.

4. Procédé selon une ou plusieurs des revendications 1 à 3, **caractérisé en ce que** le traitement avec la résine échangeuse d'ions est réalisé à des températures de 60 à 120 °C, de préférence de 60 à 90 °C.

5. Procédé selon une ou plusieurs des revendications 1 à 4, **caractérisé en ce que** le traitement avec la résine échangeuse d'ions est réalisé de la pression normale à 0,5 MPa, de préférence à la pression normale.

6. Procédé selon la revendication 1, **caractérisé en ce que** les résines échangeuses d'ions fonctionnalisées contiennent des groupes thiourée ou alkylthiourée contenant des radicaux alkyle en C1-C5.

7. Procédé selon une ou plusieurs des revendications 1 à 6, **caractérisé en ce que** la résine échangeuse d'ions est brûlée après le traitement et le rhodium est récupéré sous forme métallique ou oxydique.

8. Procédé selon une ou plusieurs des revendications 1 à 6, **caractérisé en ce que** la résine échangeuse d'ions est mise en contact après le traitement avec une solution aqueuse contenant des phosphines solubles dans l'eau de formule 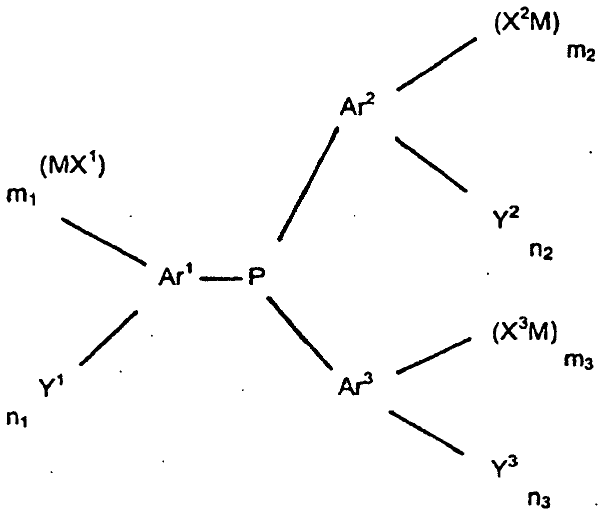 dans laquelle Ar¹, Ar² et Ar³ représentent chacun un groupe phényle ou naphtyle, Y¹, Y², Y³ représentent chacun un groupe alkyle linéaire ou ramifié de 1 à 4 atomes C, un groupe alcoxy, un atome d'halogène, un groupe OH, CN, NO₂ ou R¹R²N, dans lequel R¹ et R² sont chacun un groupe alkyle linéaire ou ramifié de 1 à 4 atomes C ; X¹, X², X³ signifient chacun un radical carboxylate (COO⁻) et/ou sulfonate (SO₃⁻) , n₁, n₂, n₃ sont des nombres entiers identiques ou différents de 0 à 5, M représente un ion de métal alcalin, l'équivalent d'un ion de métal alcalino-terreux ou de zinc ou un ion d'ammonium ou d'alkylammonium quaternaire de formule générale N(R³R⁴R⁵R⁶)⁺, dans laquelle R³, R⁴, R⁵, R⁶ sont chacun un groupe alkyle linéaire ou ramifié de 1 à 4 atomes C, et m₁, m₂, m₃ sont des nombres entiers identiques ou différents de 0 à 3, au moins un nombre m₁, m₂ ou m₃ étant supérieur ou égal à 1, en présence d'un gaz de synthèse à température élevée et pression élevée, et une solution aqueuse contenant du rhodium est récupérée.
